# EUROPÄISCHE PATENTSCHRIFT

(11) **EP 0 737 684 B1**
(45) Veröffentlichungstag und Bekanntmachung des Hinweises auf die Patenterteilung: **12.11.2003**
(21) Anmeldenummer: 96810205.3
(22) Anmeldetag: 02.04.1996
(51) Int. Cl.: C07D 403/12, C07D 249/18, C07D 249/08, C09K 15/30, C23F 11/14, C10M 133/44

(54) **(Benz)Triazolreste enthaltende Verbindungen, deren Herstellung und deren Verwendung als Metalldesaktivatoren und Korrosionsschutzmittel**
(Benzo)triazole containing compounds, their preparation and their use as metal deactivators and corrosion inhibitors
Composés contenant des groupes (benzo)triazole, leur préparation et leur utilisation comme désactivateurs de métaux et inhibiteurs de corrosion

(30) Priorität: 11.04.1995 CH 107095
(43) Veröffentlichungstag der Anmeldung: 16.10.1996
(73) Patentinhaber: Ciba Specialty Chemicals Holding Inc., 4057 Basel (CH)
(72) Erfinder: Wolf, Jean-Pierre, Dr., 1791 Courtaman (CH)

(56) Entgegenhaltungen:
- EP-A- 0 006 710
- EP-A- 0 160 620
- EP-A- 0 365 476
- EP-A- 0 385 951
- WO-A-85/01964
- DE-A- 1 521 762
- DE-A- 2 601 719
- CHEMICAL ABSTRACTS, vol. 93, no. 25, 22.Dezember 1980 Columbus, Ohio, US; abstract no. 239321n, BELOUSOV A.M. ET AL.: "Reaction of vinyl ethers with tetrazole" Seite 846; Spalte 1; XP002008837 & ZH. ORG. KHIM. (ZORKAE), Bd. 16, Nr. 6, 1980, Seiten 1313-1315, & J. ORG. CHEM. USSR (JOCYA9), Bd. 16, Nr. 6, 1980, Seiten 1135-1137,
- JOURNAL OF THE CHEMICAL SOCIETY, PERKIN TRANSACTIONS 1 (JCPRB4), Nr. 4, April 1987, Seiten 791-797, XP002008836 KATRITZKY A.R. ET AL.: "The chemistry of N-substituted benzotriazoles. Part 2. Reactions of benzotriazole with aldehydes and aldehyde derivatives. 1-(.alpha.-Hydroxyalkyl)-, 1-(.alpha.-alkoxyalkyl)-, and 1-(.alpha.-acyloxyalkyl)benzotriazoles"

## Beschreibung

Die Erfindung betrifft neue, vor allem als Metalldesaktivatoren und Korrosionsinhibitoren geeignete Verbindungen mit Triazol- und/oder Benzotriazolgruppen, deren Mischungen, diese Verbindungen oder deren Mischungen enthaltende Zusammensetzungen sowie ihre Verwendung und Herstellung.

Kupferionen katalysieren bekanntlich die Autoxidation sowie die Peroxidradikalbildung in organischen Materialien. Dies trifft auch für den oxidativen Abbau von Schmierstoffen zu. (Vgl. Ullmann's Encyclopedia of Industrial Chemistry, Vol A3, p. 104). Durch Zugabe von Benzotriazol oder Benzotriazol-Derivaten, meist zusammen mit Antioxidantien, kann die Beschleunigung der Schmierstoffzersetzung durch Kupfer drastisch verringert werden.

Man setzt technisch zur Zeit beispielsweise Verbindungen des Typs 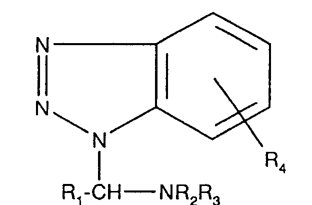 ein, worin z.B. R₁ Wasserstoff, R₂ und R₃ 2-Ethylhexyl oder Hydroxyethyl sind und R₄ Wasserstoff oder Methyl ist (s. z.B. US 4,683,071 und US 4,701,273).
In US 5,076,948 werden N-Triazolverbindungen mit Alkoxygruppen beschrieben. Weiterhin sind aus den Patentschriften US 4,153,565 und US 5,032,300 N-Benzotriazolverbindungen mit Alkoxygruppen bekannt.

Katritzky et al. beschreiben Verbindungen, welche zwei über Stickstoff gebundene Benzotriazolreste enthalten [A.R. Katritzky et al., J. Chem Soc. Perkin Trans. **1987**, 791; **1990**, 1717; J. Heterocyclic Chem. **27**, 1543 (1990)].

DE-A-15 21 762 beschreibt Verbindungen, welche zwei über die 6-Position verknüpfte Benzotriazolreste enthalten.

DE-A-26 01 719 und EP-A-0 160 620 beschreiben Verbindungen, welche zwei über eine Alkylaminoalkylbrücke verknüpfte Benzotriazolreste enthalten.

WO-A-85 01964 beschreibt Verbindungen, welche zwei über eine Alkylenbrücke verknüpfte Benzotriazolreste enthalten.

Belousov et al. (Chemical Abstracts, Vol. 93, no. 25, 22 december 1980, abstract no. 239321 n) beschreiben Verbindungen, welche zwei verknüpfte Tetrazolreste enthalten.

Es besteht weiterhin ein Bedarf an Wirkstoffen mit metalldesaktivierenden bzw. korrosionsverhindernden Eigenschaften.

Es wurde nun gefunden, daß die im folgenden näher beschriebenen, zwei (Benzo)triazolreste enthaltenden Verbindungen ausgezeichnete metalldesaktivierende und korrosionsverhindernde Eigenschaften aufweisen.

Die Erfindung betrifft daher Verbindungen der Formel I 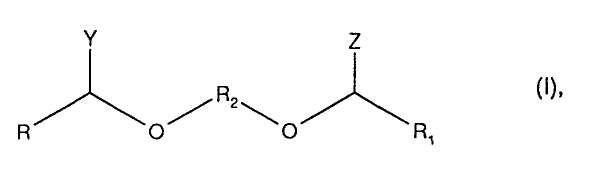 worin
Y und Z ein Rest der Formel 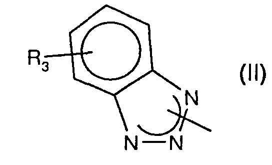 sind,
R und R₁ unabhängig voneinande C₁-C₁₂-Alkyl, bedeutet,
R₂ C₂-C₂₀ Alkylen ist,
R₃ für Wasserstoff oder C₁-C₄-Alkyl steht, sowie Mischungen solcher Verbindungen.
Die Erfindung betrifft insbesondere auch Mischungen der soeben beschriebenen Verbindungen der Formel I.

Bevorzugt sind in den Verbindungen der Formel I R und R ₁ gleich.
Bevorzugt sind ferner Verbindungen der Formel 1, worin
R₃ Wasserstoff oder Methyl ist.
Besonders bevorzugt sind in den soeben beschriebenen Verbindungen
R und R₁ Wasserstoff oder C₁-C₆-Alkyl und
R₂ C₂-C₈-Alkylen.

C₁-C₁₂-Alkyl bedeutende Reste können gerad- oder verzweigtkettig sein und sind je nach der bezeichneten Zahl der C-Atome beispielsweise Methyl, Ethyl, Propyl, Isopropyl, n-Butyl, Isobutyl, t-Butyl, Pentyl, Isopentyl, Hexyl, Heptyl, Octyl, 2-Ethylhexyl, Nonyl, Decyl, Undecyl, Dodecyl, 2-Ethylbutyl, 1-Methylpentyl, 1,3-Dimethylbutyl, 1,1,3,3-Tetramethyl butyl, 1-Methylhexyl, Isoheptyl, 1-Methylheptyl, 1,1,3-Trimethylhexyl, oder 1-Methylundecyl.

R₃ als Alkylrest hat bevorzugt 1-4 C-Atome und ist insbesondere Methyl.

Mit C₁-C₄-Alkyl substituiertes Phenyl ist vorzugsweise mit Methyl oder Ethyl substituiert und ist insbesondere Mesityl, Xylyl oder Tolyl.

Die Erfindung betrifft auch ein Verfahren zu Herstellung von Verbindungen der Formel I und deren Mischungen, bei dem man ein Benzotriazol der Formel 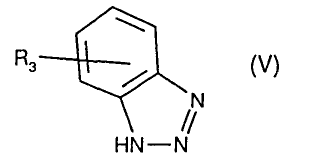 oder Mischungen davon
(i) mit Aldehyden R₁CHO oder RCHO oder Mischungen solcher Aldehyde und Diolen HO-R₂-OH umsetzt, wobei die Reste R, R₁, R₂ und R₃ wie anfangs definiert sind oder
(ii), wenn R₁ und R Methyl sind und R₂ und R₃ wie anfangs definiert sind, man mit Divinylethern der Formel H₂C=CH-O-R₂-O-CH=CH₂ umsetzt.

Der nicht festgelegte Ort der freien Bindung in Formel II 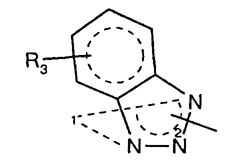 soll andeuten, daß jeweils die Substitution in 1- oder 2-Position des Benzotriazols erfolgen kann, da das Benzotriazol in mesomeren Grenzstrukturen existiert.

Für die Herstellung der Verbindungen der Formel I kommen z. B. die beiden im folgenden erläuterten und in den Beispielen ausführlich illustrierten, in der organischen Chemie geläufigen, Methoden in Frage [vgl. A.R. Katritzky et al. , J. Chem Soc. Perkin Trans. **1987**, 791; **1990**, 1717; J. Heterocyclic. Chem. **27**, 1543 (1990); J. Chem. Soc. Perkin Trans. **1989,** 639]. Man kann das Benzotriazol mit einer aequimolaren Menge Aldehyd und der halben molaren Menge eines Diols der Formel HO-R ₂-OH - beispielsweise sauer katalysiert, z.B. durch p-Toluolsulfonsäure - umsetzen. Alternativ kann das Benzotriazol direkt mit der halben molaren Menge eines Divinylethers der Formel H₂C=CH-O-R₂-O-CH=CH₂ (VI) umgesetzt werden - vorteilhaft ebenfalls sauer katalysiert. Man erhält dann Verbindungen der Formel I, worin R und R₁ Methyl sind (siehe Schema weiter unten).

Die Umsetzungen erfolgen z.B. nach folgendem Reaktionsschema:

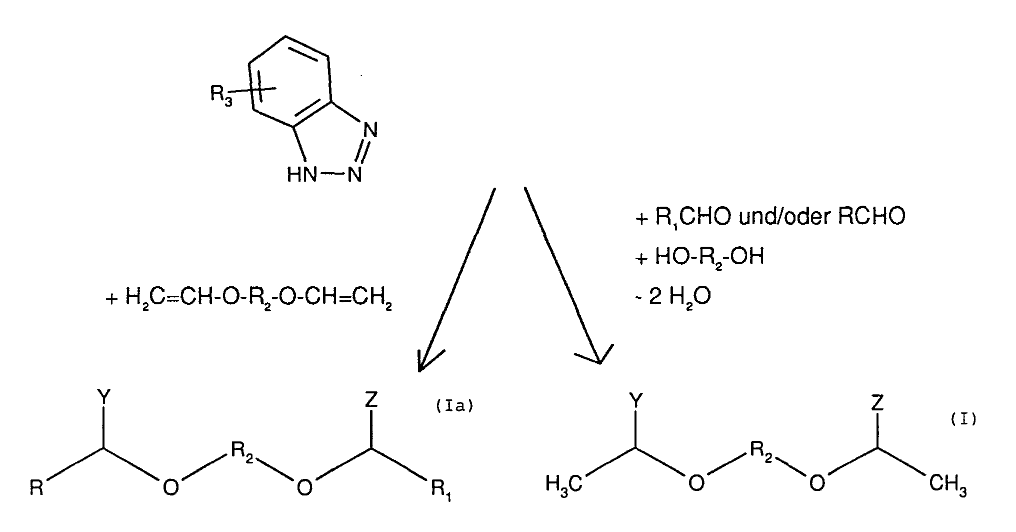

Darin haben die Reste Y, Z, R und R₁ bis R₃ die anfangs angegebenen Bedeutungen.

Die Kondensation kann in unpolaren organischen Lösungsmitteln unter Katalyse einer Säure wie zweckmäßigerweise para-Toluolsulfonsäure erfolgen.
Die Verbindungen können auch in Alkoholen oder Alkohol/Wasser-Gemischen hergestellt werden, beispielsweise in Ethanol, Methanol oder deren Mischungen mit Wasser. Unter diesen Umständen kann auf einen sauren Katalysator verzichtet werden.

Die Produkte können, wie oben erwähnt, in der 1- oder 2-Position des Benzotriazolsystems substituiert sein (1- oder 2-Benzotriazolylverbindungen). Eine Trennung etwaiger Isomeren ist nicht vonnöten, kann aber mit üblichen Methoden, wie z.B. Chromatographie, erfolgen. Bevorzugt werden in der Praxis die aus der Reaktion direkt erhaltenen Gemische eingesetzt.

Die eingesetzten Ausgangsstoffe sind kommerziell erhältlich oder nach bekannten Verfahren herstellbar. Es ist darauf hinzuweisen, daß beim Einsatz von Methylbenzotriazol bevorzugt eine Mischung von 4- und 5-Methylbenzotriazol verwendet wird.

Die erfindungsgemäßen Verbindungen eignen sich hervorragend als Metalldesaktivatoren und Antioxidantien für organische Materialien, insbesondere solche, die in Kontakt mit Metallen kommen oder Metallionen als Verunreinigungen enthalten. Für Schmiermittel zeigt sich auch eine deutliche Verschleißschutzaktivität. Die Erfindung betrifft daher auch Zusammensetzungen enthaltend
a1) einen Schmierstoff, eine Metallbearbeitungs- oder Hydraulikflüssigkeit
   oder
a2) ein Beschichtungs- oder Überzugsmittel, insbesondere einen Lack,
   und
b) mindestens eine Verbindung der Formel I, wobei die oben als bevorzugt genannten Verbindungen der Formel I und deren Gemische zu bevorzugten Zusammensetzungen führen.

Die Verbindungen der Formel I wirken an der Verhinderung von Oxidations- und Zersetzungsprozessen mit, indem sie insbesondere Kupferionen binden und so desaktivieren. Die Erfindung betrifft daher auch die Verwendung von Verbindungen der Formel I als Additive in Schmierstoffen, Hydraulik- und Metallbearbeitungsflüssigkeiten, Beschichtungs- und Überzugsmitteln, insbesondere als Metalldesaktivatoren und Korrosionsinhibitoren.

Die in Frage kommenden Schmierstoffe, Metallbearbeitungs- und Hydraulikflüssigkeiten basieren beispielsweise auf mineralischen oder syn thetischen Ölen oder Mischungen davon. Die Schmierstoffe sind dem Fachmann ge läufig und in der einschlägigen Fachliteratur, wie beispielsweise in Dieter Klamann, "Schmierstoffe und verwandte Produkte" (Verlag Chemie, Weinheim, 1982), in Schewe- Kobek, "Das Schmiermittel-Taschenbuch" (Dr. Alfred Hüthig-Verlag, Heidelberg, 1974) und in "Ullmanns Enzyklopädie der technischen Chemie", Bd.13, Seiten 85-94 (Verlag Chemie, Weinheim, 1977) beschrieben.

Die Schmierstoffe sind insbesondere Öle und Fette, beispielsweise basierend auf einem Mineralöl. Bevorzugt sind Öle.

Eine weitere Gruppe von Schmierstoffen, die zur Anwendung gelangen können, sind pflanzliche oder tierische Öle, Fette, Talge und Wachse oder deren Gemische unter einander oder Gemische mit den erwähnten mineralischen oder synthetischen Ölen. Pflanzliche und tierische Öle, Fette, Talge und Wachse sind beispielsweise Palmkernöl, Palmöl, Olivenöl, Rüböl, Rapsöl, Leinöl, Erdnußöl, Sojabohnenöl, Baumwollöl, Sonnenblumenöl, Kürbiskernöl, Kokosnußöl, Maisöl, Rizinusöl, Baumnußöl und Mischungen davon, Fischöle, Talge von Schlachttieren wie Rindertalg, Klauenfett und Knochenöl sowie deren modifizierte, epoxidierte und sulfoxidierte Formen, beispielsweise epoxi diertes Sojabohnenöl.

Die Mineralöle basieren insbesondere auf Kohlenwasserstoffverbindungen.

Beispiele von synthetischen Schmierstoffen umfassen Schmierstoffe auf der Basis der aliphatischen oder aromatischen Carboxylester, der polymeren Ester, der Polyalkylen oxide, der Phosphorsäureester, der Poly-α-olefine oder der Silicone, eines Diesters einer zweiwertigen Säure mit einem einwertigen Alkohol, wie z.B. Dioctylsebacat oder Dinonyl adipat, eines Triesters von Trimethylolpropan mit einer einwertigen Säure oder mit einem Gemisch solcher Säuren, wie z.B. Trimethylolpropantripelargonat, Trimethylol propan-tricaprylat oder Gemische davon, eines Tetraesters von Pentaerythrit mit einer ein wertigen Säure oder mit einem Gemisch solcher Säuren, wie z.B. Pentaerythrit-tetra caprylat, oder eines komplexen Esters von einwertigen und zweiwertigen Säuren mit mehrwertigen Alkoholen, z.B. ein komplexer Ester von Trimethylolpropan mit Capryl- und Sebacinsäure oder von einem Gemisch davon. Besonders geeignet sind neben Mineral ölen z.B. Poly-α-Olefine, Schmierstoffe auf Esterbasis, Phosphate, Glykole, Poly glykole und Polyalkylenglykole, sowie deren Mischungen mit Wasser.

Metallbearbeitungsflüssigkeiten und Hydraulikflüssigkeiten können auf Basis der gleichen Substanzen hergestellt werden wie vorstehend für die Schmiermittel beschrieben. Häufig handelt es sich dabei auch um Emulsionen solcher Substanzen in Wasser oder anderen Flüssigkeiten.

Erfindungsgemäße Schmierstoffzusammensetzungen finden z.B. Verwendung in Ver brennungsmotoren, z.B. in Kraftfahrzeugen, ausgerüstet z.B. mit Motoren des Otto-, Diesel-, Zweitakt-, Wankel- oder Orbitaltyps.

Die Verbindungen der Formel I sind gut in Schmierstoffen, Metallbearbeitungs- und Hydraulikflüssigkeiten löslich und sind deshalb als Zusätze zu Schmierstoffen, Metallbearbeitungs- und Hydraulikflüssigkeiten besonders geeignet.

Die Verbindungen der Formel I bzw. deren Gemische können den Schmierstoffen auf an sich bekannte Weise beigemischt werden. Die Verbindungen sind beispielsweise in Ölen gut löslich. Es ist auch möglich, einen sogenannten Masterbatch herzustellen, der nach Maßgabe des Verbrauchs auf Einsatzkonzentrationen mit dem entsprechenden Schmierstoff verdünnt werden kann. In solchen Fällen sind auch Konzentrationen über 10 Gew.% möglich.

Die erfindungsgemäßen Verbindungen, wie oben beschrieben, können z.B. in Mengen von 0,01 bis 10 Gew.-%, zweckmäßig in Mengen von 0,01 bis 5 Gew.-%, vorzugsweise in einer Menge von 0,01 bis 3 Gew.-% und ganz besonders bevorzugt von 0,01 bis1,5 Gew.-%, bezogen auf die Zusammensetzung, in dem Schmierstoff, der Metallbearbeitungs- oder der Hydraulikflüssigkeit vorliegen.

Zusätzlich zu den erfindungsgemäßen Verbindungen können die Schmiermittel, Metallbearbeitungs- und Hydraulikflüssigkeiten noch weitere übliche Additive enthalten, wie z.B. weitere Antioxidantien, Metall desaktivatoren, Rostinhibitoren, Viskositäts-Index--Verbesserer, Stockpunkterniedriger, Dispergiermittel/Tenside und Hochdruck- und Verschleißschutzadditive (Extreme Pressure/Anti Wear Additives).

Beispiele hierfür sind:

### Beispiele für phenolische Antioxidantien

1. Alkylierte Monophenole, z.B. 2,6-Di-tert-butyl-4-methylphenol, 2-Tert-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tertbutyl-4-methoxymethylphenol, 2,6-Di-nonyl-4-methyl phenol, 2,4- Dimethyl-6-(1'-methylundec-1'-yl)-phenol, 2,4-Dimethyl-6-(1'-methyl-heptadec-1'-yl)-phenol, 2,4-Dimethyl-6-(1'methyl-tridec-1'-yl)-phenol und Mischungen davon.
2. Alkylthiomethylphenole, z.B. 2,4-Di-octylthiomethyl-6-tert-butylphenol, 2,4-Di- octylthiomethyl-6-methylphenol, 2,4-Di-octylthiomethyl-6-ethylphenol, 2,6-Di-do decylthiomethyl-4-nonylphenol.
3. Hydrochinone und alkylierte Hydrochinone, z.B. 2,6-Di-tert-butyl-4-methoxyphenol, 2,5--Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5- Di-tert-butyl-4-hydroxyanisol, 3,5-Di-tert-butyl-4-hydroxyphenyl-stearat, Bis- (3,5-di-tert-butyl-4-hydroxyphenyl)adipat.
4. Tocopherole, z.B. α-Tocopherol, β-Tocopherol, γ-Tocopherol, δ-Tocopherol und Mischungen davon (Vitamin E).
5. Hydroxylierte Thiodiphenylether, z.B. 2,2'-Thio-bis-(6-tert-butyl-4-methylphenol), 2,2'--Thio-bis-(4-octylphenol), 4,4'-Thio-bis-(6-tert-butyl-3-methylphenol), 4,4'-Thio-bis-(6-tertbutyl-2-methylphenol), 4,4'-Thio-bis-(3,6-di-sec.-amylphenol), 4,4'-Bis-(2,6-dimethyl-4-hydroxyphenyl)-disulfid.
6. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(a-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tertbutylphenol), 2,2'-Ethyliden-bis-(6-tert-butyl-4-isobutylphenol), 2,2'-Methylen-bis-[6-(amethylbenzyl)-4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1 -Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1 -Bis-(5-tert-butyl-4-hydroxy-2-methyl-phenyl)-3-n-dodecylmercaptobutan, Ethylen glycol-bis-[3,3-bis-(3'-tert-butyl-4'-hydroxyphenyl)-butyrat], Bis-(3-tert-butyl-4-hydroxy-5-methylphenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-ditert-butyl-4-hydroxyphenyl)-propan, 2,2-Bis-(5-tert-butyl-4-hydroxy-2-methylphenyl)-4-ndodecylmercapto-butan, 1,1,5,5-Tetra-(5-tert-butyl-4-hydroxy-2-methylphenyl)-pentan . 7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, lsooctyl-3,5-di-tert-butyl-4-hydroxybenzylmercaptoacetat.
8. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.
9. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6--Tris-(3,5-di-tert-butyl-4-hydroxybenzyl)-phenol.
10. Triazinverbindungen, z.B. 2,4-Bis-octylmercapto-6-(3,5-di-tert-butyl-4-hydroxy anilino)--1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy) -1,3,5-triazin, 2,4,6- Tris-(3,5-ditert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-di methylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro- 1,3,5-triazin, 1,3,5-Tris-(3,5-dicyclohexyl-4-hydroxybenzyl)-isocyanurat.
11. Benzylphosphonate, z.B. Dimethyl-2,5-di-tert-butyl-4-hydroxybenzylphosphonat, Di ethyl-3,5-di-tert-butyl-4-hydroxybenzylphosphonat, Dioctadecyl-3,5-di-tert-butyl-4- hydroxybenzylphosphonat, Dioctadecyl-5-tert-butyl-4-hydroxy-3-methyl benzylphosphonat, Ca-Salz des 3,5-Di-tert-butyl-4-hydroxybenzyl-phosphonsäure-monoethylest ers.
12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.
13. Ester der β-(3.5-Di-tert-butyl-4-hydroxyphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Tri methylhexandiol, Trimethylolpropan, 4-Hydroxymethyl-1-phospha-2,6,7-trioxabicyclo-[2.2.2]-octan.
14. Ester der β-(5-tert-Butyl-4-hydroxy-3-methylphenyl)-propionsäure mit ein- oder mehrwertigen Alkoholen, wie z.B. mit Methanol, Ethanol, Octanol, Octadecanol, 1,6-Hexan diol, 1,9-Nonandiol, Ethylenglycol, 1,2-Propandiol, Neopentyl glycol, Thiodiethylenglycol, Diethylenglycol, Triethylenglycol, Pentaerythrit, Tris-(hydroxy)ethyl-isocyanurat, N,N'-Bis--(hydroxyethyl)-oxalsäurediamid, 3-Thiaundecanol, 3-Thiapentadecanol, Tri methylhexandiol, Trimethylolpropan, 4-Hydroxymethyl-1-phospha-2,6,7-trioxabicyclo-[2.2.2]-octan.
15. Ester der β-(3.5-Dicyclohexyl-4-hydroxyphenyl ) -propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentyl glycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Tri methylhexandiol, Trimethylolpropan, 4-Hydroxymethyl-1-phospha-2,6,7-tri oxabicyclo-[2.2.2]-octan.
16. Ester der 3,5-Di -tert -butyl -4 -hydroxyphenylessigsäure mit ein- oder mehrwertigen Alkoholen, wie z.B. mit Methanol, Ethanol, Octanol, Octadecanol, 1,6-Hexandiol, 1,9- Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Tri methylhexandiol, Trimethylolpropan, 4-Hydroxymethyl-1-phospha-2,6,7-tri oxabicyclo-[2.2.2]-octan.
17. Amide der β-(3.5-Di -tert -butyl -4-hydroxyphenyl)-propionsäure, wie z.B. N,N'-Bis-(3,5-ditert-butyl-4-hydroxyphenyl propionyl)-hexamethylendiamin, N,N'-Bis-(3,5-di- tert-butyl-4-hydroxyphenylpropionyl)-trimethylendiamin, N,N'-Bis-(3,5-di-tert-butyl-4-hydroxyphenylpropionyl)-hydrazin.

### Beispiele für aminische Antioxidantien:

N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylen diamin, N,N'-Bis(1-methyl-heptyl)-p-phenylendiamin, N,N'-Dicyclohexyl-p-phenylen diamin, N,N'-Diphenylp-phenylendiamin, N,N'-Di-(naphthyl-2)-p-phenylendiamin, N-Iso propyl-N'-phenyl-pphenylendiamin, N-(1,3-Dimethyl-butyl)-N'-phenyl-p- phenylendiamin, N-(1-Methyl-heptyl)-N'phenyl-p-phenylendiamin, N-Cyclohexyl-N'-phenyl-p-phenylendiamin, 4-(p-Toluolsulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylaminophenol, 4-Octadecanoylamino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Isopropyl/lsohexyl-diphenylaminen, Gemische aus mono- und dialkylierten tert-Butyldiphenylaminen, 2,3-Dihydro-3,3-dimethyl-4H-1,4-benzothiazin, Phenothiazin, N-Allylphenothiazin, N,N,N',N'--Tetraphenyl-1,4-diaminobut-2-en, N,N-Bis-(2,2,6,6-tetramethyl-piperidin-4-yl)-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.

### Beispiele für weitere Antioxidantien:

Aliphatische oder aromatische Phosphite, Ester der Thiodipropionsäure oder der Thio diessigsäure, oder Salze der Dithiocarbamid- oder Dithiophosphorsäure, 2,2,12,12-Tetramethyl-5,9-dihydroxy-3,7,11-trithiatridecan und 2,2,15,15-Tetramethyl-5,12-dihydroxy--3,7,10,14-tetrathiahexadecan.

### Beispiele für Metall-Desaktivatoren, z.B. für Kupfer, sind:

a) Benzotriazole und deren Derivate, z.B. 4- oder 5-Alkylbenztriazole (z.B. Tolutriazol) und deren Derivate, 4,5,6,7-Tetrahydrobenztriazol, 5,5'-Methylenbis-benztriazol; Man nich--Basen von Benztriazol oder Tolutriazol wie 1-[Di(2-ethylhexyl)aminomethyl]-tolutriazol und 1-[Di(2-ethylhexyl)aminomethyl]-benztriazol; Alkoxyalkylbenztriazole wie 1-(Nonyloxymethyl)-benztriazol, 1-(1-Butoxyethyl)-benztriazol und 1-(1-Cyclohexyloxybutyl)-tolutriazol.
b) 1,2,4-Triazole und deren Derivate, z.B. 3-Alkyl (oder Aryl)- 1,2,4-Triazole, Man nich--Basen von 1,2,4-Triazolen wie 1-[Di(2-ethylhexyl)amino methyl]-1,2,4-triazol; Alkoxyalkyl-1,2,4-triazole wie 1-(1-Butoxyethyl)-1,2,4-triazol; acylierte 3-Amino-1,2,4-triazole.
c) Imidazolderivate, z.B. 4,4'-Methylenbis(2-undecyl)-5-methylimidazol, Bis[(N-methyl)-imidazol-2-yl]carbinol-octylether.
d) Schwefelhaltige heterocyclische Verbindungen, z.B. 2-Mercaptobenzthiazol, 2,5-Dimercapto-1,3,4-thiadiazol und deren Derivate; 3,5-Bis[di(2- ethylhexyl)amino-methyl]-1,3,4-thiadiazolin-2-on.
e) Aminoverbindungen, z.B. Salicyliden-propylendiamin, Salicylaminoguanidin und deren Salze.

### Beispiele für Rost-Inhibitoren sind:

a) Organische Säuren, ihre Ester, Metallsalze, Aminsalze und Anhydride, z.B. Alkylund Alkenylbernsteinsäuren und deren Partialester mit Alkoholen, Diolen oder Hy droxycarbonsäuren, Partialamide von Alkyl- und Alkenylbernsteinsäuren, 4-No nylphenoxyessigsäure, Alkoxy- und Alkoxyethoxycarbonsäuren wie Dodecy loxyessigsäure, Dodecyloxy(ethoxy)-essigsäure und deren Aminsalze, ferner N-Oleoyl-sarcosin, Sorbitanmono-oleat, Blei-naphthenat, Alkenylbernsteinsäure anhydride, z.B. Dodecenylbernsteinsäure-anhydrid, 2-Carboxymethyl-1-dodecyl-3-methylglycerin und dessen Aminsalze.
b) Stickstoffhaltige Verbindungen, z.B.:
   I. Primäre, sekundäre oder tertiäre aliphatische oder cycloaliphatische Amine und Aminsalze von organischen und anorganischen Säuren, z.B. öllösliche Alkyl ammoniumcarboxylate, ferner 1-[N,N-bis-(2-hydroxyethyl)amino]-3-(4-nonylphenoxy)propan-2-ol.
   II. Heterocyclische Verbindungen, z.B.:
      Substituierte Imidazoline und Oxazoline, 2-Heptadecenyl-1-(2-hydroxyethyl)- imidazolin.
c) Phosphorhaltige Verbindungen, z.B.:
   Aminsalze von Phosphorsäurepartialestern oder Phosphonsäurepartialestern, Zinkdialkyldithiophosphate.
d) Schwefelhaltige Verbindungen, z.B.:
   Barium-dinonylnaphthalin-sulfonate, Calciumpetroleum-sulfonate, Alkylthio- substituierte aliphatische Carbonsäuren, Ester von aliphatischen 2-Sulfocarbonsäuren und deren Salze.
e) Glycerinderivate, z.B.:
   Glycerin-monooleat, 1-(Alkylphenoxy)-3-(2-hydroxyethyl)glycerine, 1-(Alkylphenoxy)-3-(2,3-dihydroxypropyl)glycerine, 2-Carboxyalkyl-1,3-dialkylglycerine.

### Beispiele für Viskositätsindex-Verbesserer sind:

Polyacrylate, Polymethacrylate, Vinylpyrrolidon/Methacrylat-Copolymere, Polyvinyl pyrrolidone, Polybutene, Olefin-Copolymere, Styrol/Acrylat-Copolymere, Polyether.

### Beispiele für Stockpunkterniedriger sind:

Polymethacrylat, alkylierte Naphthalinderivate.

### Beispiele für Dispergiermittel/Tenside sind:

Polybutenylbernsteinsäureamide oder -imide, Polybutenylphosphonsäurederivate, basische Magnesium-, Calcium-, und Bariumsulfonate und -phenolate.

### Beispiele für Verschleissschutz-Additive sind:

Schwefel und/oder Phosphor und/oder Halogen enthaltende Verbindungen, wie ge schwefelte Olefine und pflanzliche Oele, Zinkdialkyldithiophosphate, alkylierte Triphenyl phosphate, Tritolylphosphat, Tricresylphosphat, chlorierte Paraffine, Alkyl- und Aryldi- und tri- sulfide, Aminsalze von Mono- und Dialkylphosphaten, Aminsalze der Methylphos phonsäure, Diethanolaminomethyltolyltriazol, Di(2-ethylhexyl)aminomethyltolyltriazol, Derivate des 2,5-Dimercapto-1,3,4-thiadiazols, 3-[(Bis-isopropyloxy-phosphinothioyl)thio]-propionsäure--ethylester, Triphenylthiophosphat (Triphenylphosphorothioat), Tris (alkylphenyl)phosphorothioate und deren Gemische, (z.B. Tris(isononyl phenyl)phosphorothioat), Diphenylmonononylphenyl-phosphorothioat, Isobutylphenyl- diphenyl-phosphorothioat, Dodecylaminsalz des 3-Hydroxy-1,3-thiaphosphetan-3-oxids, Tri thiophosphorsäure-5,5,5-tris[isooctylacetat (2)], Derivate von 2-Mercaptobenzthiazol wie 1-[N,N- Bis(2-ethylhexyl)aminomethyl]-2-mercapto-1H-1,3-benzthiazol, 5-Ethoxycarbonyloctyl-dithiocarbamat.

Besonders wirksam sind die erfindungsgemäßen Verbindungen zusammen mit phenolischen und/oder aminischen Antioxidantien.

Überzugsmittel bestehen im allgemeinen aus Bindemitteln, Zusatzstoffen und gegebenenfalls farbgebenden Komponenten.

Als Bindemittel kommen prinzipiell alle in der Technik gebräuchlichen in Betracht, beispielsweise solche, wie sie beschrieben sind in Ullmann's Encyclopedia of Industrial Chemistry, 5. Ed., Vol. A18, pp. 368-426, VCH, Weinheim 1991. Allgemein handelt es sich um ein filmbildendes Bindemittel basierend auf einem thermoplastischen oder thermohärtbaren Harz, vorwiegend auf einem thermohärtbaren Harz. Beispiele hierfür sind Alkyd-, Acryl-, Polyester-, Phenol-, Melamin-, Epoxid-, Polyurethanharze und deren Gemische.

Es kann sich um ein kalt aushärtbares oder ein heiß aushärtbares Bindemittel handeln, wobei die Zugabe eines Härtungskatalysators vorteilhaft sein kann. Geeignete Katalysatoren, die die Aushärtung des Bindemittels beschleunigen, sind bei spielsweise beschrieben in Ullmann's Encyclopedia of Industrial Chemistry, Vol. A18, S.469, VCH Verlagsgesellschaft, Weinheim 1991.

Bevorzugt sind Überzugsmittel, die als filmbildendes Bindemittel Epoxidharze, Polyurethanharze, Polyesterharze, Acrylharze und deren Copolymerharze, Polyvinylharze, Phenolharze, Alkydharze oder Mischungen solcher Harze enthalten.
Die Verbindungen der Formel I können in den Überzugsmitteln zu 0,001 bis 10, bevorzugt zu 0,1 bis 5 % enthalten sein.

Handelt es sich bei den erfindungsgemäßen Zusammensetzungen um Überzugsmittel bzw. Lacke, so können diese ebenfalls weitere übliche Komponenten enthalten, z.B. aus der Gruppe der Farbstoffe, Pigmente, Füllstoffe, Fließkontrollmittel, Haftverbesserer, Härtungskatalysatoren, Lichtschutzmittel oder Antioxidantien.

Bevorzugte erfindungsgemäße Verbindungen, wie vorstehend beschrieben, führen zu bevorzugten Zusammensetzungen.

Die vorliegende Erfindung betrifft auch die Verwendung von Verbindungen oder Verbindungsgemischen der Formel I als Additive in Schmierstoffen, Hydraulik- und Metallbearbeitungsflüssigkeiten oder Beschichtungs- und Überzugsmitteln sowie ein Verfahren zur Verbesserung der Gebrauchseigenschaften von Schmierstoffen, Metallbearbeitungs- oder Hydraulikflüssigkeiten oder von Beschichtungs- oder Überzugsmitteln, dadurch gekennzeichnet, daß diesen Verbindungen oder Verbindungsgemische der Formel I zugesetzt werden.

Die folgenden Beispiele erläutern die Erfindung weiter, ohne sie jedoch zu beschränken. Teile- und Prozentangaben beziehen sich, sofern nicht anders angegeben, auf das Gewicht. Es wird jeweils unter Stickstoff gearbeitet. Wenn die Produkte bereits beim Abkühlen der Reaktionslösung zu kristallisieren beginnen, wird abfiltriert und auf weitere Reinigung verzichtet.

### Beispiele

### Herstellungsbeispiele

(In den Tabellen sowie in der übrigen Beschreibung bedeuten Me = Methyl.

### Methode 1

In einem 4-Hals-Sulfierkolben (Reaktionsgefäß mit flachem Boden) mit mechanischem Rührer, Wasserabscheider nach Dean-Stark, Thermometer und Stick stoffeinleitung werden 0,2 mol Benzotriazol, 0,2mol Aldehyd, 0,1mol Diol (vgl. Tabelle1) und 0,2g p-Toluolsulfonsäure in 200ml Cyclohexan vorgelegt.

Die Reaktionslösung wird so lange am Rückfluß erhitzt, bis sich die theoretische Menge von 0,2mol Wasser abgeschieden hat.

Die auf Raumtemperatur abgekühlte Reaktions lösung wird dreimal mit 100ml wäßriger 5% Na₂CO₃-Lösung und mit zweimal 100ml Wasser gewaschen, über MgSO₄ getrocknet und das Lösungsmittel am Rotationsverdampfer abdestilliert. Zum Schluß wird 2 Stunden bei 60°C im Hochvakuum getrocknet.

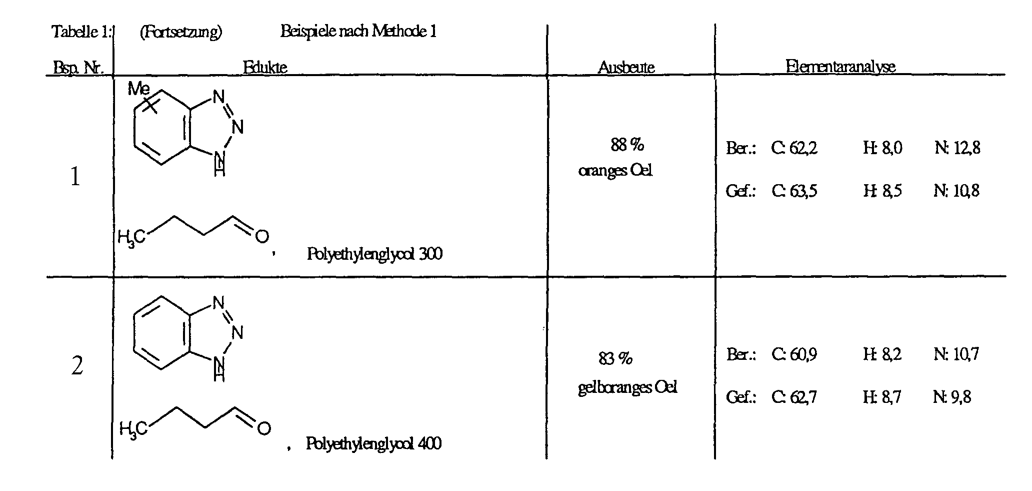

### Methode 2

In einem 4-Hals-Sulfierkolben mit mechanischem Rührer, Rückflußkühler, Thermometer und Stickstoffeinleitung werden 0,2mol Triazol, 0,1mol Divinylether und 0,2g p-Toluolsulfonsäure in 100ml Lösungsmittel (vgl. Tabelle2) vorgelegt.

Die Reaktionslösung wird bis zum Verschwinden der Edukte (Kontrolle durch Dünnschichtchromatographie) am Rückfluß gekocht.

Die auf Raumtemperatur abgekühlte Reaktionslösung wird dreimal mit 100ml wäßriger 5% Na₂CO₃-Lösung und zweimal mit 100ml Wasser gewaschen, über MgSO₄ getrocknet und das Lösungsmittel am Rotationsverdampfer abdestilliert. Zum Schluß wird 2Stunden bei 60°C im Hochvakuum getrocknet.

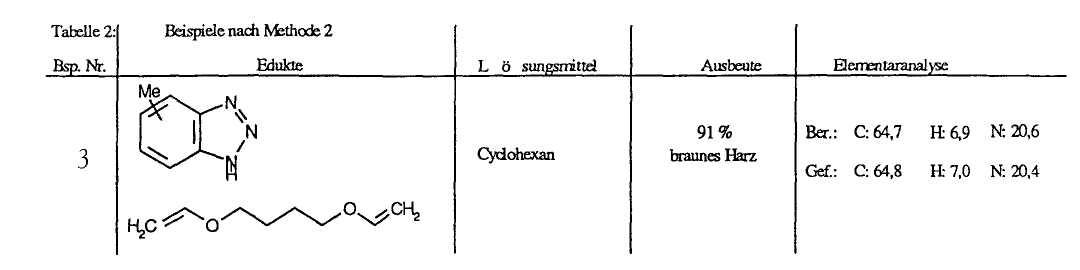

### Methode 3

In einem 4-Hals-Sulfierkolben mit mechanischem Rührer, Rückflußkühler, Thermometer und Stickstoffeinleitung werden 0,2mol Benzotriazol, 0,1mol Divinylether und 0,2g p-Toluolsulfonsäure in 100ml Lösungsmittel (vgl. Tabelle3) vorgelegt.

Die Reaktionslösung wird bis zum Verschwinden der Edukte (Kontrolle durch Dünnschichtchromatographie) am Rückfluß gekocht. Zu der auf Raumtemperatur abgekühlten Reaktionslösung gibt man 5g pulverisiertes CaO und etwas MgSO₄, läßt 10Minuten rühren und filtriert die Suspension. Das Filtrat wird am Rotations verdampfer eingeengt und das Produkt 2Stunden bei 60 °C im Hochvakuum getrocknet.

### Methode 4

In einem 4-Hals-Sulfierkolben mit mechanischem Rührer, Wasser abscheider nach Dean-Stark, Thermometer und Stickstoffeinleitung werden 0,2mol Benzotriazol, 0,2mol Butyraldehyd, 0,1mol 1,4-Bis(hydroxymethyl)-cyclohexan und 0,2g p-Toluolsulfonsäure in einem Gemisch von 100ml Cyclohexan und 100ml Toluol zum Rückfluß erhitzt.

Nachdem sich die theoretische Menge von 0,2mol Wasser abgetrennt hat, wird auf Raumtemperatur abgekühlt. Es werden 5g CaO und etwas MgSO₄ zugegeben, 10Minuten rühren gelassen und die Suspension filtriert. Das Filtrat wird am Rotations verdampfer eingeengt und das Produkt 2Stunden bei 60 °C im Hochvakuum getrocknet.

### Anwendungsbeispiele

### Beispiel A1: Kupfer Korrosionstest (modifiziert nach ASTM D-130)

0,05 Gewichts% der zu testenden Verbindung werden in einem Turbinenöl der Viskosität 29,7 mm²s⁻¹ bei 40°C und 5,05 mm²s⁻¹ bei 100°C (Schwefelgehalt 0,22%) gelöst. Weitere 50 ppm elementarer Schwefel werden zugegeben.

Ein mit Siliciumcarbid poliertes Kupferblech (60 x 10 x 1 mm) wird vollständig in die Öllösung getaucht und 3 Stunden bei 100 °C dort belassen. Danach wird das Kupferblech aus dem Öl genommen und mit Petrolether gespült. Es folgt die Bewertung nach der ASTM D 130 Copper Strip Corrosion Standard Chart (Vgl. Tabelle 5). Die Beurteilung geschieht in vier Stufen:
1 - kein Beschlag 2 - mäßiger Beschlag
3 - starker Beschlag 4 - Korrosion,
wobei innerhalb der Zahlengruppen 1 bis 4 noch auf Grund der Schattenbildung auf den Proben eine Feinunterteilung vorgenommen wird. In der qualitativen Beurteilung A bis E steht dabei die Wertung A vor B, B vor C usw.

### Beispiel A2: Rotary Bomb Oxidation Test (RBOT), ASTM D 2272

0,05 Gewichts% der zu testenden Verbindung werden in einem Turbinenöl (Viskosität 29,7 mm²s⁻¹ bei 40°C und 5,05 mm² s⁻¹ bei 100°C, Schwefelgehalt 0,22%) gelöst. Weitere Komponenten sind
0,15% eines phenolischen¹⁾, 0,05% eines aminischen²⁾ Antioxidans und 0,07% eines Korrosionsinhibitors³⁾ 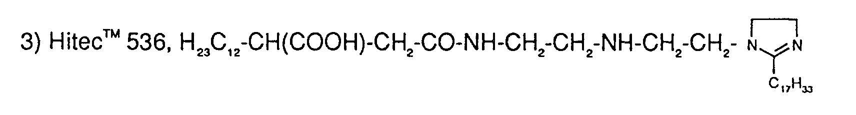 (Vgl. folgende Tabelle . 50 ml der so erhal tenen Mischung werden mit 5 ml Wasser in das Testgefäß gegeben, das als Katalysator eine Kupferspirale enthält. Das Gefäß wird mit Sauerstoff bis zu einem Druck von 620 kPa beladen, verschlossen und in einem 150°C heißen Bad rotiert. Gemessen wird die Zeit, in welcher der Sauerstoffdruck um 172 kPa abfällt.
1) Gemisch von tert-butylierten Phenolen, erhältlich als Irganox™ 140
2) Gemisch von Diphenylamin-Verbindungen, kommerziell erhältlich als Irganox ™-57, vgl. US-5,073,278, Sp. 2, Z. 50

## Patentansprüche

1. Verbindungen der Formel I 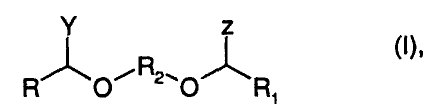 worin Y und Z einen Rest der Formel 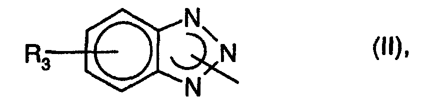 worin R und R₁ C₁-C₁₂-Alkyl, R₂ C₂-C₂₀Alkylen und R₃ Wasserstoff oder C₁-C₄Alkyl bedeuten, sowie Mischungen dieser Verbindungen.

2. Verbindungen oder Mischungen gemäss Anspruch 1, worin R und R₁ gleich sind.

3. Verbindungen oder Mischungen gemäss Anspruch 1, worin R und R₁ C₁-C₉-Alkyl, R₂ C₂-C₁₅-Alkylen und R₃ Wasserstoff oder Methyl bedeuten.

4. Verbindungen oder Mischungen gemäss Anspruch 1, worin R und R₁ C₁-C₆Alkyl, R₂ C₂-C₁₂-Alkylen und R₃ Wasserstoff oder Methyl bedeuten.

5. Verbindungen oder Mischungen gemäss Anspruch 4, worin R₂ C₂-C₈-Alkylen bedeutet.

6. Verfahren zur Herstellung von in Anspruch I definierten Verbindungen der Formel I und deren Mischungen, **dadurch gekennzeichnet, dass** man ein Benztriazol der Formel 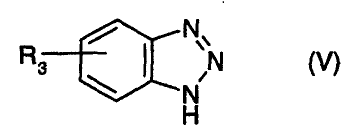
(i) mit Aldehyden R₁CHO oder RCHO oder Mischungen solcher Aldehyde und Diole HO-R₂-OH umsetzt, wobei die Reste R, R₁, R₂ und R₃ wie in Anspruch 1 definiert sind, oder
(ii) wenn R und R₁ Methyl sind und R₂ und R₃ wie in Anspruch 1 definiert sind, mit Divinylethern der Formel H₂C=CH-O-R₂-O-CH=CH₂ umsetzt.

7. Zusammensetzungen enthaltend
(A) einen Schmierstoff, eine Hydraulik- oder Metallbearbeitungsflüssigkeit oder ein Beschichtungs- oder Überzugsmittel und
(B) mindestens eine Verbindung oder ein Gemisch von Verbindungen (I) nach Anspruch 1.

8. Zusammensetzungen nach Anspruch 7, worin die Komponente A) ein Schmierstoff ist.

9. Zusammensetzungen nach Anspruch 8, worin der Schmierstoff ein Motoröl ist.

10. Zusammensetzungen nach Anspruch 7, die zusätzlich Antioxidantien, Metalldesaktivatoren, Rostinhibitoren, Viskositätsindexverbesserer, Stockpunkterniedriger, Dispergiermittel, Tenside, Hochdruckadditive oder Verschleissschutzadditive enthalten.

11. Zusammensetzungen nach Anspruch 10, die als weitere Antioxidantien phenolische oder aminische Antioxidantien enthalten.

12. Verfahren zur Verbesserung der Gebrauchseigenschaften von Schmierstoffen, Hydraulikoder Metallbearbeitungsflüssigkeiten oder ein Beschichtungs- oder Überzugsmittel, **dadurch gekennzeichnet, dass** diesen Verbindungen gemäss Anspruch 1 oder Zusammensetzungen gemäss Anspruch 7 zugesetzt werden.

## Claims

1. A compound of the formula I 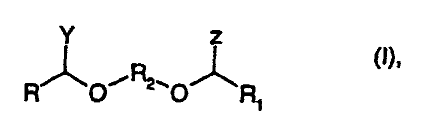 in which Y and Z are a radical of the formula 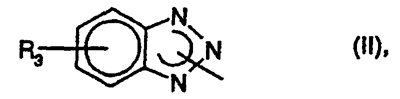 in which R and R₁ are C₁-C₁₂alkyl, R₂ is C₂-C₂₀alkylene and R₃ is hydrogen or C₁-C₄alkyl, or a mixture of such compounds.

2. A compound or mixture according to claim 1, wherein R and R₁ are identical.

3. A compound or mixture according to claim 1, wherein R and R₁ are C₁-C₉alkyl, R₂ is C₂-C₁₅alkylene and R₃ is hydrogen or methyl.

4. A compound or mixture according to claim 1, wherein R and R₁ are C₁-C₆alkyl, R₂ is C₂-C₁₂alkylene and R₃ is hydrogen or methyl.

5. A compound or mixture according to claim 4, wherein R₂ is C₂-C₈alkylene.

6. A process for the preparation of compounds of the formula I defined in claim 1 and mixtures thereof, which comprises reacting a benzotriazole of the formula 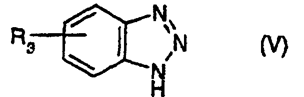
(i) with aldehydes R₁CHO or RCHO or mixtures of such aldehydes and diols HO-R₂-OH, in which the radicals R, R₁, R₂ and R₃ are as defined in claim 1, or
(ii) if R and R₁ are methyl and R₂ and R₃ are as defined in claim 1, with divinyl ethers of the formula H₂C=CH-O-R₂-O-CH=CH₂.

7. A composition comprising
(A) a lubricant, a hydraulic or metalworking fluid or a coating composition and
(B) at least one compound or mixture of compounds (I) according to claim 1.

8. A composition according to claim 7, wherein component A) is a lubricant.

9. A composition according to claim 8, wherein the lubricant is an engine oil.

10. A composition according to claim 7, which additionally comprises antioxidants, metal deactivators, rust inhibitors, viscosity index improvers, pour-point depressants, dispersants, surfactants, extreme pressure additives or antiwear additives.

11. A composition according to claim 10 which comprises as further antioxidants phenolic or amine-type antioxidants.

12. A method for improving the service properties of lubricants, hydraulic or metalworking fluids or a coating composition which comprises adding thereto compounds according to claim 1 or compositions according to claim 7.

## Revendications

1. Composés de formule I 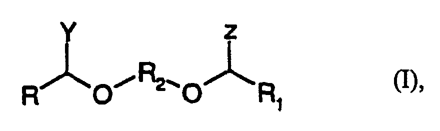 dans laquelle Y et Z représentent un reste de formule 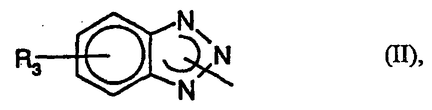 R et R₁ représentent un reste alkyle en C₁-C₁₂, R₂ représente un reste alkylène en C₂-C₂₀ et R₃ représente un atome d'hydrogène ou un reste alkyle en C₁-C₄, et des mélanges de ces composés.

2. Composés ou mélanges selon la revendication 1, dans lesquels R et R₁ sont identiques.

3. Composés ou mélanges selon la revendication 1, dans lesquels R et R₁ représentent un reste alkyle en C₁-C₉, R₂ représente un reste alkylène en C₂-C₁₅ et R₃ représente un atome d'hydrogène ou un reste méthyle.

4. Composés ou mélanges selon la revendication 1, dans lesquels R et R₁ représentent un reste alkyle en C₁-C₆, R₂ représente un reste alkylène en C₂-C₁₂ et R₃ représente un atome d'hydrogène ou un reste méthyle.

5. Composés ou mélanges selon la revendication 4, dans lesquels R₂ représente un reste alkylène en C₂-C₈.

6. Procédé de préparation de composés de formule I définis dans la revendication 1 et de leurs mélanges, **caractérisé en ce que** l'on fait réagir un benzotriazole de formule 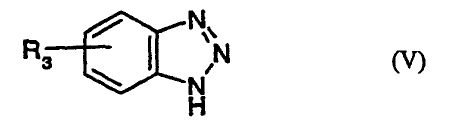
(i) avec des aldéhydes R₁CHO ou RCHO ou des mélanges de ces aldéhydes et des diols HO-R₂-OH, les restes R, R₁, R₂ et R₃ ayant la définition donnée dans la revendication 1, ou
(ii) lorsque R et R₁ sont des restes méthyle et R₂ et R₃ ont la définition donnée dans la revendication 1, avec des éthers divinyliques de formule H₂C=CH-O-R₂-O-CH=CH₂.

7. Compositions contenant
(A) un lubrifiant, un fluide hydraulique, un fluide de traitement des métaux ou un produit de peinture ou de revêtement et
(B) au moins un composé ou un mélange de composés (I) selon la revendication 1.

8. Compositions selon la revendication 7, dans lesquelles le constituant (A) est un lubrifiant.

9. Compositions selon la revendication 8, dans lesquelles le lubrifiant est une huile de moteur.

10. Compositions selon la revendication 7, contenant en outre des antioxydants, des désactiveurs de métaux, des inhibiteurs de corrosion, des agents améliorant l'indice de viscosité, des agents abaissant le point d'écoulement, des dispersants, des agents tensioactifs, des additifs haute pression ou des additifs de protection contre l'usure.

11. Compositions selon la revendication 10, contenant comme autres antioxydants des antioxydants phénoliques ou aminés.

12. Procédé d'amélioration des propriétés d'emploi de lubrifiants, de fluides hydrauliques, de fluides de traitement des métaux ou d'une composition de peinture ou de revêtement, **caractérisé en ce qu'**on leur ajoute des composés selon la revendication 1 ou des compositions selon la revendication 7.
